(19) Europäisches Patentamt
European Patent Office
Office européen des brevets

(11) Veröffentlichungsnummer: **0 070 804**
**B1**

(12) # EUROPÄISCHE PATENTSCHRIFT

(45) Veröffentlichungstag der Patentschrift: **08.04.87**

(21) Anmeldenummer: **82810300.2**

(22) Anmeldetag: **12.07.82**

(51) Int. Cl.[4]: **C 07 D 239/46**, C 07 D 239/52, C 07 D 239/56, C 07 D 239/48, C 07 D 251/46, A 01 N 47/36

(54) **Fluoralkoxy-aminopyrimidine und -triazine.**

(30) Priorität: **16.07.81 CH 4667/81**
**06.08.81 CH 5075/81**
**13.10.81 CH 6541/81**
**11.01.82 CH 124/82**
**08.04.82 CH 2205/82**
**08.06.82 CH 3527/82**

(43) Veröffentlichungstag der Anmeldung: **26.01.83 Patentblatt 83/4**

(45) Bekanntmachung des Hinweises auf die Patenterteilung: **08.04.87 Patentblatt 87/15**

(84) Benannte Vertragsstaaten: **AT BE CH DE FR GB IT LI NL**

(56) Entgegenhaltungen:
**EP-A-0 007 687**
**EP-A-0 013 480**
**EP-A-0 030 139**

(73) Patentinhaber: **CIBA- GEIGY AG, Klybeckstrasse 141, CH- 4002 Basel (CH)**

(72) Erfinder: **Meyer, Willy, Talweg 49, CH- 4125 Riehen (CH)**

**EP 0 070 804 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die vorliegende Erfindung betrifft neue Fluoralkoxyaminopyrimidine und -triazine, Verfahren zu ihrer Herstellung und ihre Verwendung bei der Synthese von Herbiziden aus der Klasse der Sulfonylharnstoffe sowie als Zwischenprodukte hergestellte N-Fluoralkoxypyrimidinyl- und -triazinylcarbamate.

Die erfindungsgemässen Fluoralkoxy-aminopyrimidine und -triazine haben die allgemeine Formel 1

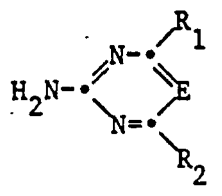

(I) ,

worin

E Stickstoff oder die Methingruppe,

$R_1$ Halogen, $C_1$-$C_4$-Alkyl, $C_1$-$C_4$-Alkoxy, $C_1$-$C_4$-Halogenalkoxy, $C_1$-$C_4$-Alkylthio, $C_1$-$C_4$-Halogenalkyl, $-NR_3R_4$ oder Alkoxyalkyl mit höchstens 4 Kohlenstoffatomen und

$R_2$ einen Rest $-G-CF_2-T$

bedeuten, wobei G für Sauerstoff und T für Wasserstoff, -CHClF, -CHBrF, $-CHF_2$ oder $-CHF-CF_3$, $R_3$ für Wasserstoff, Methyl oder Aethyl und $R_4$ für Wasserstoff, Methyl, Aethyl, Methoxy, Aethoxy oder Methoxymethyl stehen, mit der Massgabe, dass $R_1$ nicht $C_1$-$C_4$-Alkyl bedeutet, wenn gleichzeitig E für Stickstoff steht.

Die erfindungsgemässen neuen Fluoralkoxy-aminopyrimidine und -triazine sind Zwischenprodukte bei der Synthese von verschiedenen Herbiziden und Pflanzenwuchsregulatoren aus der Klasse der Sulfonylharnstoffe. Solche Sulfonylharnstoffe sind in den Europäischen Patentanmeldungen EP-A- 70802, EP-A 72347, EP-A- 82108 und EP-A- 84020 beschrieben.

Sulfonylharnstoffe mit herbizider Wirkung sind schon lange aus der Literatur bekannt. Beispielsweise werden sie im US Patent 4 127 405 oder den Europäischen Patentanmeldungen EP-A- 7687, EP-A- 13480 und EP-A- 30139 beschrieben

Halogenalkoxytriazine werden in der DE-OS 2 013 424 als Pflanzenschutzmittel hervorgehoben.

Unter Halogen ist im Rahmen der obigen Definition im allgemeinen Fluor, Chlor, Brom oder Jod zu verstehen. Bevorzugt ist Fluor oder Chlor, insbesondere aber Fluor.

Beispiele für Alkyl sind Methyl, Aethyl, n-Propyl, i-Propyl oder die isomeren Butyl. Dabei ist Alkyl als Substituent oder als Teil eines anderen Substituenten wie z.B. Alkoxy, Halogenalkoxy, Alkylthio, Halogenalkyl, oder Alkoxyalkyl zu verstehen. Bevorzugt sind unverzweigte Alkylketten, insbesondere aber Methyl und Aethyl.

Die Gruppe der Verbindungen der Formel 1 zerfällt in zwei grosse Untergruppen, die gleichermassen bevorzugt sind: diese Untergruppen enthalten diejenigen Verbindungen, in denen

a) $R_1$ Halogen, $C_1$-$C_4$-Alkylthio, $C_1$-$C_4$-Alkoxy, $C_1$-$C_4$-Alkyl, $C_1$-$C_4$-Halogenalkoxy, $C_1$-$C_4$-Halogenalkyl oder Alkoxyalkyl mit höchstens 4 Kohlenstoffatomen bedeutet und diejenigen Verbindungen, in denen

b) $R_1$ die Gruppe $-NR_3R_4$ bedeutet, worin $R_3$ für Wasserstoff, Methyl der Aethyl und $R_4$ für Wasserstoff, Methyl, Aethyl, Methoxy, Aethoxy oder Methoxymethyl stehen.

Als weiterhin bevorzugte Untergruppen sind zu nennen: Verbindungen der Formel 1, in denen

c) $R_2$ für 1,1,2,2-Tetrafluoräthoxy steht und in denen

d) $R_2$ für $-G-CHF_2$ steht, worin G Sauerstoff bedeutet.

Eine besonders bevorzugte Untergruppe der Verbindungen der Gruppe c) bilden die Verbindungen, in denen

$R_1$ Fluor, Chlor, Methyl, Aethyl, Methoxy, Aethoxy, Difluormethoxy, Trifluormethyl, Fluormethyl, Methoxymethyl oder $-NR_3R_4$ bedeutet, wobei $R_3$ für Wasserstoff oder Methyl und $R_4$ für Methyl oder Aethyl steht.

Eine besonders bevorzugte Untergruppe der Verbindungen der Gruppe d) bilden die Verbindungen, in denen

E die Methingruppe, $R_1$ Fluor, Chlor, Methyl, Aethyl, Methoxy, Aethoxy, Difluormethoxy, Trifluormethyl, Fluormethyl, Methoxymethyl oder $-NR_3R_4$ bedeutet, wobei $R_3$ für Wasserstoff oder Methyl und $R_4$ für Methyl oder Methyl bedeuten.

Hiervon bilden eine weitere hervorzuhebenden Gruppe die Verbindungen, in denen

$R_1$ Methyl, Aethyl, Methoxy, Aethoxy, Difluormethoxy, Dimethylamino oder Aethylmethylamino bedeuten.

Die Verbindungen der Formel I werden hergestellt, indem man eine Aminoverbindung der Formel II

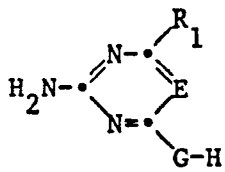

(II) ,

worin E und $R_1$ die unter Formel I angegebene Bedeutung haben und G für Sauerstoff steht, in Gegenwart einer Base mit Difluorchlormethan, Tetrafluoräthylen, Trifluorchloräthylen, Trifluorbromäthylen, Hexafluorpropylen, oder Difluorbrommethan umsetzt.

Das Verfahren zur Herstellung der Verbindungen der Formel I wird mit Vorteil in einem inerten polaren Lösungsmittel oder Lösungsmittelgemisch ausgeführt. Geeignete Lösungsmittel sind Aether wie Dioxan, Tetrahydrofuran, Aethylenglykoldimethyläther, Diäthylenglykoldimethyläther; Alkohole wie Methanol, Aethanol, Isopropanol; Ketone wie Aceton, Aethylmethylketon; Dimethylformamid; Acetonitril oder Dimethylsulfoxid. Als Basen sind in besonderer Weise geeignet: Natrium- und Calciumhydrid, Kalium- und Natriumhydroxid, Kalium- und Natriumcarbonat. In geeigneten Fällen kann die Base als wässrige Lösung zugesetzt werden.

Bei der Verwendung von gasförmigen Fluorkohlenwasserstoffen als Ausgangsmaterialien zur Herstellung der Verbindungen der Formel I kann es von Vorteil sein, wenn man die Reaktion unter erhöhtem Druck durchführt. Es können dabei Drücke bis zu 100 Bar, vorzugsweise bis zu 20 Bar angewendet werden.

Die Reaktionstemperaturen betragen im allgemeinen 0° bis 120° C, vorzugsweise 20° bis 100° C.

In einzelnen Fällen ist es von Vorteil, die Reaktion in Gegenwart von Phasen-Transfer-Katalysatoren durchzuführen, z.B. von quaternären Ammoniumverbindungen oder Kronenäthern.

Die erfindungsgemässen Fluoralkoxy-aminopyrimidine und -triazine werden in die herbiziden Sulfonylharnstoffe aus den Europäischen Patentanmeldungen EP-A 70802, EP-A 72347, EP-A 82108 und EP-A-84020 überführt, indem man

a) das Fluoralkoxy-aminopyrimidin oder -triazin der Formel I gegebenenfalls in Gegenwart einer Base mit einem Sulfonylisocyanat oder -isothiocyanat der Formel III

$X\text{-}SO_2\text{-}N=C=Z$ (III),

worin X einen in den genannten Patentanmeldungen umfassten aromatischen Rest bedeutet und Z für Sauerstoff oder Schwefel steht, umsetzt oder

b) das Fluoralkoxy-aminopyrimidin oder -triazin der Formel I mit einem Carbamat der Formel IV

$$X\text{-}SO_2\text{-}NH\text{-}\overset{\overset{\displaystyle Z}{\|}}{C}\text{-}O\text{-}C_6H_5 \qquad (IV) \quad ,$$

worin X einen in den genannten Patentanmeldungen umfassten aromatischen Rest bedeutet und Z für Sauerstoff oder Schwefel steht, umsetzt oder

c) das Fluoralkoxy-aminopyrimidin oder -triazin der Formel I zunächst in ein Isocyanat oder Isothiocyanat der Formel V

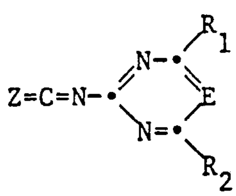

(V) ,

worin E, $R_1$ und $R_2$ die unter Formel I gegebene Bedeutung haben und für Sauerstoff oder Schwefel steht, überführt und gegebenenfalls in Gegenwart einer Base dann mit einem Sulfonamid der Formel VI

$X\text{-}SO_2\text{-}NH_2$ (VI),

worin X einen in den genannten Patentanmeldungen umfassten aromatischem Rest bedeutet, umsetzt oder

d) das Fluoralkoxy-aminopyrimidin oder -triazin der Formel I zunächst in ein N-Fluoralkoxypyrimidinyl- oder -triazinylcarbamat der Formel VII

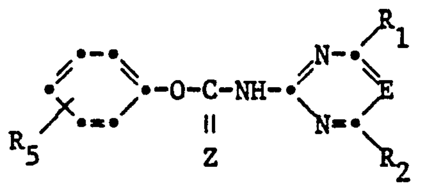

(VII)

worin E, $R_1$ und $R_2$ die unter Formel I gegebene Bedeutung haben, Z für Sauerstoff oder Schwefel steht und $R_5$ ein Substituent aus der Reihe Wasserstoff, Halogen, Nitro oder $C_1$-$C_3$-Alkyl ist, überführt und dann in Gegenwart einer Base mit einem Sulfonamid der oben angegebenen Formel VI umsetzt.

Die Verwendung der erfindungsgemässen Verbindungen der Formel I zur Synthese von Herbiziden aus der Klasse der Sulfonylharnstoffe bildet ebenfalls einen Gegenstand der vorliegenden Erfindung.

Die Ausgangsmaterialien der Formeln III, IV und VI sind bekannt, in den genannten Patentanmeldungen beschrieben oder können nach bekannten Methoden hergestellt werden.

Isocyanate und Isothiocyanate der Formel V sind neu und können analog zu bekannten Verfahren aus den erfindungsgemässen Verbindungen der Formel I hergestellt werden. Aehnliche Reaktionen sind in "Neuere Methoden der präparativen organischen Chemie", Band VI, 211-223, Verlag Chemie, Weinheim 1970, bzw. Arch.Pharm. 299, 174 (1966) beschrieben.

N-Fluoralkoxypyrimidinyl- und -triazinylcarbamate der Formel VII sind neu und speziell für die Ueberführung der Verbindungen der Formel I unter milden Reaktionsbedingungen in die herbiziden Sulfonylharnstoffe der genannten Patentanmeldungen geschaffen worden. Sie bilden deshalb einen weiteren Aspekt der vorliegenden Erfindung.

Die Verbindungen der Formel VII werden hergestellt, indem man Kohlensäurederivate der Formel VIII

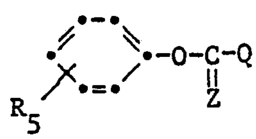

(VIII) ,

worin

Z und $R_5$ wie unter Formel VII definiert sind und Q für Chlor, Brom oder durch $R_5$ substituiertes Phenoxy steht, gegebenenfalls in Gegenwart einer Base mit einem Amin der Formel I umsetzt.

Das Verfahren zur Herstellung der Verbindungen der Formel VII wird mit Vorteil in einem inerten aprotischen Lösungsmittel bei Temperaturen zwischen 20° und 120°C durchgeführt. Geeignete Lösungsmittel sind Aether wie Diäthyläther, Dioxan, Tetrahydrofuran, Aethylenglykoldimethyläther, Diäthylenglykoldimethyläther; Ketone wie Aceton, Aethylmethylketon, Cyclohexanon; Kohlenwasserstoffe wie Benzol, Toluol, Xylol, Cyclohexan; chlorierte Kohlenwasserstoffe wie Methylenchlorid, Chloroform, Tetrachlorkohlenstoff; Dimethylformamid; N-Methylpyrrolidinon; Acetonitril oder Dimethylsulfoxid. Als Basen sind z.B. besonders geeignet: tertiäre Amine wie Triäthylamin, Chinuclidin, Chinolin, Pyridin, Trimethylamin, 4-Dimethylaminopyridin, Carbonate wie Natrium- oder Kaliumcarbonat oder Hydride wie Natrium- oder Calciumhydrid. Vorteilhafterweise werden die Basen dabei im Ueberschuss verwendet.

In einzelnen Fällen kann das Amin der Formel I im Ueberschuss eingesetzt werden, und bei der Reaktion so als Base dienen.

Die folgenden Beispiele dienen der näheren Erläuterung der vorliegenden Erfindung.

**Beispiel 1**: 2-Amino,4-difluormethoxy-6-methyl-pyrimidin

In eine Lösung von 62,5 g 2-Amino-4-hydroxy-6-methyl-pyrimidin in 500 ml Wasser, 100 ml 40 %iger Natriumhydroxidlösung und 100 ml Dioxan wird über einem Zeitraum von 12 Stunden bei einer Temperatur von 70 bis 75°C gasförmiges Difluorchlormethan eingeleitet. Während dieses Zeitraumes fügt man, jeweils im Abstand von 1 Stunde, in gleichen Portionen insgesamt 160 g festes Natriumhydroxid zu. Durch Abtrennen der organischen Phase, Einengen auf ca. 1/10 des Volumens, Eingiessen des Rückstandes in Wasser und Abtrennen des ausgefallenen Feststoffes erhält man 39,9 g 2-Amino-4-difluormethoxy-6-methyl-pyrimidin, Smp. 136 bis 137°C (Verbindung Nr. 1.1).

**Beispiel 2**: 2-Amino-4,6-bis-(difluormethoxy)-pyrimidin.

Analog zu Beispiel 1 erhält man aus 63,5 g 2-Amino-4,6-dihydroxy-pyrimidin durch Einleiten von 105 g Difluorchlormethan innerhalb von 6 Stunden 7,3 g 2-Amino-4,6-bis-(difluormethoxy)-pyrimidin, Smp. 63 bis 65°C (Verbindung Nr. 1.11).

**Beispiel 3**: 2-Amino-4-chlor-6-difluormethoxy-pyrimidin.

Eine Suspension von 16,4 g 2-Amino-4,6-dichlor-pyrimidin in 100 ml 40 %iger Natriumhydroxidlösung wird für 1 Stunde bei einer Temperatur von 95 bis 100°C gerührt. Nach Zusatz von 200 ml Dioxan werden bei einer Temperatur von 70 bis 75°C innerhalb von 1 Stunde 20 g gasförmiges Difluormethan eingeleitet. Durch Abtrennen der organischen Phase, Einengen auf ca. 1/5 des Volumens, Eingiessen in Wasser und Abtrennen des festen Niederschlages erhält man 6 g 2-amino-4-chlor-6-difluormethoxy-pyrimidin, Smp. 118 bis 119°C (Verbindung Nr. 1.7)

**Beispiel 4**: 2-Amino-4-difluormethoxy-6-methoxy-pyrimidin.

19,2 g 2-Amino-4,6-dimethoxy-pyrimidin-hydrochlorid werden während 2 Stunden auf 150°C erhitzt, wobei sich unter Abspaltung von Methylchlorid 2-Amino-4-hydroxy-6-methoxy-pyrimidin bildet. Nach Zusatz von 80 ml 40 %iger Natriumhydroxidlösung und 100 ml Dioxan werden bei 70 bis 75° während 3/4 Stunden 22 g Difluorchlormethan eingeleitet. Durch Abtrennen der organischen Phase, Einengen auf ca. 1/5 des Volumens, Eingiessen in Wasser und Abtrennen des festen Niederschlages erhält man 2,4 g 2-Amino-4-difluormethoxy-6-methoxypyrimidin, Smp. 106 bis 107°C (Verbindung Nr. 1.7).

**Beispiel 5**: 2-amino-4-methyl-6-[1,1,2-trifluor-2-chlor-

-äthoxylpyrimidin.

25 g 2-Amino-4-hydroxy-6-methyl-pyrimidin, 25,6 g Chlortrifluoräthylen, 13,8 g Kaliumhydroxid und 200 ml Dimethylformamid werden zusammen während 8 Stunden bei 60° im Autoklaven gerührt. Durch Verdünnen mit Wasser, Extrahieren mit Essigester und Eindampfen erhält man ein dunkles Oel. Nach der Zugabe von wenig Methylenchlorid und Abtrennen des gebildeten kristallinen Niederschlages erhält man 8,7 g 2-Aamino-4-methyl-6-(1,1,2-trifluor-2-chlor-äthoxy)-pyrimidin, Smp. 73 bis 74°C (Verbindung Nr. 1.42).

**Beispiel 6**: N-(2-Difluormethoxyphenyl-sulfonyl)-N'-(4-difluormethoxy-

-6-methyl-pyrimidin-2-yl)-harnstoff.

Eine Mischung aus 2,5 g 2-Difluormethoxyphenyl-sulfonylisocyanat, 1,75 g 2-Amino-difluormethoxy-6-methyl-pyrimidin und 30 ml absolutem Dioxan wird für 2 Stunden bei einer Temperatur von 70 bis 75°C gerührt. Durch Eindampfen der Lösung und Kristallisation des Rückstandes aus Aether erhält man 4,0 g N-(2-Difluormethoxyphenyl-sulfonyl)-N'-(4-difluormethoxy-6-methyl-pyrimidin-2-yl)-harnstoff, Smp. 163 bis 164°C.

**Beispiel 7**: N-(2-Methoxycarbonylphenyl-sulfonyl)-N'-[4,6-bis-(difluormethoxy)-pyrimidin-2-yl]-harnstoff.

Analog zu Beispiel 6 erhält man aus 3,62 g 2-Methoxycarbonyl-sulfonyl-isocyanat und 2,63 g 2-Amino-4,6-bis-(difluormethoxy)-pyrimidin in 50 ml Dioxan 3,9 g N-(2-Methoxycarbonylphenyl-sulfonyl)-N'-[4,6-bis-(difluormethoxy)-pyrimidin-2-yl]-harnstoff, Smp. 186 bis 188°C.

**Beispiel 8**: N-[2-(2-Aethoxy-äthoxy)-phenyl- sulfonyl]-N'-(4-difluormethoxy-6-methyl-pyrimidin-2-yl)-harnstoff.

a) Einer Lösung von 9,0 g 2-Amino-4-difluormethoxy-6-methyl-pyrimidin in 30 ml absolutem Tetrahydrofuran werden nacheinander 3,9 g Chlorameisensäure-phenylester und 0,05 g 4-Dimethylamino-pyridin zugesetzt.

Nach Rühren der Lösung bei 20 bis 25°C für 24 Stunden wird das Reaktionsgemisch mit 250 ml Aethylacetat verdünnt und der ausgefallene Niederschlag abgetrennt. Man erhält so 3,1 g 2-Amino-4-difluormethoxy-6-methyl-pyrimidin-hydrochlorid, Smp. 204 bis 205°C (Zers.). Das Filtrat wird zur Entfernung des restlichen Ausgangsstoffes über eine Säure mit Ionenaustauscher I® (MERCK) eluiert, über Natriumsulfat getrocknet und eingedampft. Man erhält so in Form eines viskosen Oeles als Rohprodukt 4,0 g 2-Phenoxycarbonyl-amino-4-difluormethoxy-6-methyl-pyrimidin (Verbindung Nr. 2.28), das nach der Kristallisation bei 56 bis 58°C schmilzt. $^1$H-NMR ($CDCl_3$): = 2,5 (s,$CH_3$), 6,45 (s,1H), 7,0-7,5 (5H), 7,56 (t,J=70 Hz, $CHF_2$) und 9,0 (NH ppm).

b) Eine Lösung von 3,5 g 2-Phenoxycarbonylamino-4-difluormethoxy-6-methyl-pyrimidin-Rohprodukt und 2,9 g 2-(2-Aethoxy-äthoxy)-benzol-sulfonamid in 100 ml absolutem Acetonitril wird mit 1,8 g 1,5-Diazabicyclo[5.4.0]undec-5-en zersetzt, für 1 Stunde bei 20 bis 25°C gerührt, dann mit 500 ml Wasser verdünnt und mit Salzsäure angesäuert. Das abgeschiedene Oel wird mit 200 ml Aethylacetat aufgenommen über Natriumsulfat getrocknet und eingedampft. Durch Kristallisieren des öligen Rückstandes aus Diäthyläther und Aceton erhält man als farbloses Kristallisat N-[2-(2-Aethoxy-äthoxy)-phenyl-sulfonyl]-N'-(4-difluormethoxy-6-methyl-pyrimidin-2-yl)-harnstoff, Smp. 90 bis 95°C (Zers.), Ausbeute 2,7 g.

In analoger Weise erhält man die in den anschliessenden Tabellen aufgelisteten Verbindungen der Formel I und daraus hergestellten Zwischenprodukte zur Synthese von Sulfonylharnstoff-Herbiziden.

**Tabelle 1**

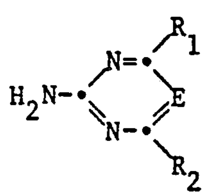

| Verb. Nr. | $R_1$ | $R_2$ | E | Smp. [°C] |
|---|---|---|---|---|
| 1.1 | $-CH_3$ | $-OCHF_2$ | CH | 136 - 137° |
| 1.2 | $-CH_2F$ | $-OCHF_2$ | CH | |
| 1.3 | $-CH_2CH_3$ | $-OCHF_2$ | CH | 76 - 77° |
| 1.4 | $-CH_2OCH_3$ | $-OCHF_2$ | CH | |
| 1.5 | $-CF_3$ | $-OCHF_2$ | CH | 95 - 98° |
| 1.6 | -Cl | $-OCHF_2$ | CH | 118 - 119° |
| 1.7 | $-OCH_3$ | $-OCHF_2$ | CH | 106 - 107° |
| 1.8 | $-OCH_2CF_3$ | $-OCHF_2$ | CH | |
| 1.9 | $-OCH_2CH_3$ | $-OCHF_2$ | CH | |
| 1.10 | $-OCH(CH_3)_2$ | $-OCHF_2$ | CH | |
| 1.11 | $-OCHF_2$ | $-OCHF_2$ | CH | 63 - 65° |
| 1.12 | $-SCH_3$ | $-OCHF_2$ | CH | |
| 1.17 | $-OCH_3$ | $-OCHF_2$ | N | |
| 1.18 | $-N(CH_3)_2$ | $-OCHF_2$ | CH | 99 - 100° |

**Tabelle 1** (Fortsetzung)

| Verb. Nr. | $R_1$ | $R_2$ | E | Smp. [°C] |
|---|---|---|---|---|
| 1.20 | $-CH_3$ | $-O-CF_2-CHF_2$ | CH | 102 - 103° |
| 1.21 | $-CH_3$ | $-O-CF_2-CHFBr$ | CH | |
| 1.22 | $-CH_3$ | $-O-CF_2-CHF-CF_3$ | CH | |
| 1.23 | $-OCH_3$ | $-O-CF_2-CHFCl$ | CH | 56 - 57° |
| 1.24 | $-OCH_3$ | $-O-CF_2-CHF_2$ | CH | |
| 1.25 | $-OCH_3$ | $-O-CF_2-CHF-CF_3$ | CH | |
| 1.26 | $-C_2H_5$ | $-O-CF_2-CHF_2$ | CH | |
| 1.27 | $-C_2H_5$ | $-O-CF_2-CHFCl$ | CH | |
| 1.28 | $-Cl$ | $-O-CF_2-CHF_2$ | CH | |
| 1.29 | $-Cl$ | $-O-CF_2-CHFCl$ | CH | 59 - 60° |
| 1.30 | $-SCH_3$ | $-O-CF_2-CHF_2$ | N | |
| 1.31 | $-SCH_3$ | $-O-CF_2-CHFCl$ | N | |
| 1.32 | $-SCH_3$ | $-O-CF_2-CHFBr$ | N | |
| 1.33 | $-SCH_3$ | $-O-CF_2-CHF-CF_3$ | N | |
| 1.34 | $-OCH_3$ | $-O-CF_2-CHF_2$ | N | |
| 1.35 | $-OCH_3$ | $-O-CF_2-CHFCl$ | N | |
| 1.36 | $-OCH_3$ | $-O-CF_2-CHFBr$ | N | |
| 1.37 | $-OCH_3$ | $-O-CF_2-CHF-CF_3$ | N | |
| 1.38 | $-OC_2H_5$ | $-O-CF_2-CHF_2$ | N | |
| 1.39 | $-OCH_2CF_3$ | $-O-CF_2-CHF_2$ | N | |
| 1.40 | $-SCH_3$ | $-O-CF_2-CHFCl$ | CH | |
| 1.41 | $-SCH_3$ | $-O-CF_2-CHF_2$ | CH | |
| 1.42 | $-CH_3$ | $-O-CF_2-CHFCl$ | CH | 73 - 74° |
| 1.44 | $-NHCH_3$ | $-OCHF_2$ | CH | |
| 1.45 | $-N(CH_3)OCH_3$ | $-OCHF_2$ | CH | |
| 1.46 | $-N(CH_3)C_2H_5$ | $-OCHF_2$ | CH | |
| 1.47 | $-N(CH_3)_2$ | $-OCF_2-CHF_2$ | CH | |

Tabelle 2

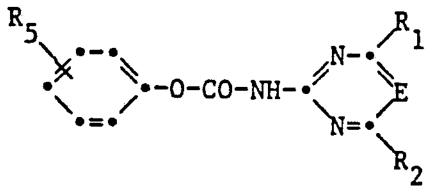

| Verb. Nr. | $R_1$ | $R_2$ | $R_5$ | E | phys. Daten |
|---|---|---|---|---|---|
| 2.1 | $-C_2H_5$ | $-OCHF_2$ | H | CH | |
| 2.2 | $-OCH_3$ | $-OCHF_2$ | H | CH | |
| 2.3 | $-OCHF_2$ | $-OCHF_2$ | H | CH | |
| 2.4 | -Cl | $-OCHF_2$ | H | CH | |
| 2.6 | $-CH_3$ | $-OCF_2-CHF_2$ | H | CH | |
| 2.7 | $-OCH_3$ | $-OCF_2-CHF_2$ | H | CH | |
| 2.8 | $-OCH_2-CF_3$ | $-OCF_2-CHF_2$ | H | CH | |
| 2.9 | -Cl | $-OCF_2-CHF_2$ | H | CH | |
| 2.10 | $-N(CH_3)_2$ | $-OCF_2-CHF_2$ | H | CH | |
| 2.11 | $-OCH_3$ | $-OCF_2-CHF_2$ | H | N | |
| 2.12 | $-CH_3$ | $-OCF_2-CHClF$ | H | N | |
| 2.13 | $-CH_3$ | $-OCF_2-CHF_2$ | H | N | |
| 2.14 | $-OCH_3$ | $-OCF_2-OHClF$ | H | CH | |
| 2.15 | $-C_2H_5$ | $-OCF_2-CHF_2$ | H | CH | |
| 2.16 | $-CF_3$ | $-OCHF_2$ | H | CH | |
| 2.17 | $-CH_2F$ | $-OCHF_2$ | H | CH | |
| 2.18 | $-CF_3$ | $-OCF_2-CHF_2$ | H | CH | |
| 2.19 | $-OCH_2-CF_3$ | $-OCHF_2$ | H | CH | |
| 2.20 | $-CH_3$ | $-OCF_2-CHClF$ | H | CH | |

**Patentansprüche**

1. Fluoralkoxy-aminopyrimidine und -triazine der allgemeinen Formel I

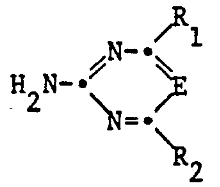

(I) ,

worin
E Stickstoff oder die Methingruppe,
$R_1$ Halogen, $C_1$-$C_4$-Alkyl, $C_1$-$C_4$-Alkoxy, $C_1$-$C_4$-Halogenalkoxy, $C_1$-$C_4$-Alkylthio, $C_1$-$C_4$-Halogenalkyl, $-NR_3R_4$ oder Alkoxyalkyl mit höchstens 4 Kohlenstoffatomen und
$R_2$ einen Rest $-G-CF_2T$
bedeuten, wobei G für Sauerstoff und T für Wasserstoff, -CHClF, -CHBrF, $-CHF_2$ oder $-CHF-CF_3$, $R_3$ für Wasserstoff, Methyl oder Aethyl und $R_4$ für Wasserstoff, Methyl, Aethyl, Methoxy, Aethoxy oder Methoxymethyl stehen, mit der Massgabe, dass $R_1$ nicht $C_1$-$C_4$-Alkyl bedeutet, wenn gleichzeitig E für Stickstoff steht.

2. Verbindungen gemäss Anspruch 1, dadurch gekennzeichnet, dass $R_1$ Halogen, $C_1$-$C_4$-Alkyl, $C_1$-$C_4$-Alkoxy, $C_1$-$C_4$-Halogenalkoxy, $C_1$-$C_4$-Alkyl-thio, $C_1$-$C_4$-Halogenalkyl oder Alkoxyalkyl mit höchstens 4 Kohlenstoffatomen bedeutet.

3. Verbindungen gemäss Anspruch 1, dadurch gekennzeichnet, dass $R_1$ die Gruppe $-NR_3R_4$ bedeutet, worin $R_3$ für Wasserstoff, Methyl oder Aethyl und $R_4$ für Wasserstoff, Methyl, Aethyl, Methoxy, Aethoxy oder Methoxymethyl stehen.

4. Verbindungen gemäss Anspruch 1 dadurch gekennzeichnet, dass $R_2$ für 1,1,2,2-Tetrafluoräthoxy steht.

5. Verbindungen gemäss Anspruch 1, dadurch gekennzeichnet, dass $R_2$ für $-G-CHF_2$ steht, worin G Sauerstoff bedeutet.

6. Verbindungen gemäss Anspruch 4, dadurch gekennzeichnet, dass $R_1$ Fluor, Chlor, Methyl, Aethyl, Methoxy, Aethoxy, Difluormethoxy Trifluormethyl, Fluormethyl, Methoxymethyl oder $-NR_3R_4$ bedeutet, wobei $R_3$ für Wasserstoff oder Methyl und $R_4$ für Methyl Aethyl steht.

7. Verbindungen gemäss Anspruch 5, dadurch gekennzeichnet, dass E die Methingruppe, $R_1$ Fluor, Chlor, Methyl, Aethyl, Methoxy, Aethoxy, Difluormethoxy, Trifluormethyl, Fluormethyl, Methoxymethyl oder $-NR_3R_4$ bedeutet, wobei $R_3$ für Wasserstoff oder Methyl und $R_4$ für Methyl oder Aethyl bedeuten.

8. Verbindungen gemäss Anspruch 7, dadurch gekennzeichnet, dass $R_1$ Methyl, Aethyl, Methoxy, Aethoxy, Difluormethoxy, Dimethylamino oder Aethylmethylamino bedeuten.

9. Verfahren zur Herstellung der Verbindungen der Formel I, gemäss Anspruch 1, dadurch gekennzeichnet, dass man eine Aminoverbindung der Formel II

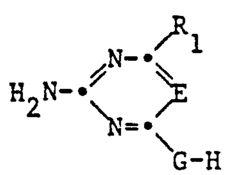

(II) ,

worin E und $R_1$ die unter Formel I angegebene Bedeutung haben und G für Sauerstoff steht, in Gegenwart einer Base mit Difluorchlormethan, Tetrafluoräthylen, Trifluorchloräthylen, Trifluorbromäthylen, Hexafluorpropylen, oder Difluorbrommethan umsetzt.

10. Verwendung der Fluoralkoxy-aminopyrimidine und -triazine der Formel I, gemäss Anspruch 1, zur Herstellung von Herbiziden aus der Klasse der Sulfonylharnstoffe.

11. N-Fluoralkoxypyrimidinyl- und -triazinyl-carbamate der allgemeinen Formel VII

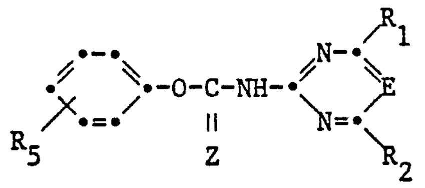 (VII) ,

worin $R_1$, $R_2$ und E die unter Formel I im Anspruch 1 gegebene Bedeutung haben, Z für Sauerstoff oder Schwefel steht und $R_5$ ein Substituent aus der Reihe Wasserstoff, Halogen, Nitro oder $C_1$-$C_3$-Alkyl bedeutet.

**Claims**

1. A fluoroalkoxy-aminopyrimidine or -triazine of the general formula I

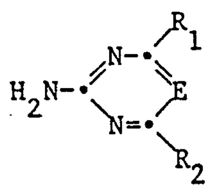 (I)

wherein
E is nitrogen or the methine group,
$R_1$ is halogen, $C_1$-$C_4$-alkyl, $C_1$-$C_4$-alkoxy, $C_1$-$C_4$-haloalkoxy, $C_1$-$C_4$-alkylthio, $C_1$-$C_4$-haloalkyl, $-NR_3R_4$ or alkoxy alkyl having at most 4 carbon atoms, and
$R_2$ is a group $-G-CF_2-T$,
where G is oxygen, T is hydrogen, -CHClF, -CHBrF, $-CHF_2$ or $-CHF-CF_3$, $R_3$ is hydrogen, methyl or ethyl, and $R_4$ is hydrogen, methyl, ethyl, methoxy, ethoxy or methoxymethyl, with the proviso that $R_1$ is not $C_1$-$C_4$-alkyl when simultaneously E is nitrogen.

2. A compound according to claim 1, wherein $R_1$ is halogen, $C_1$-$C_4$-alkyl, $C_1$-$C_4$-alkoxy, $C_1$-$C_4$-haloalkoxy, $C_1$-$C_4$-alkylthio, $C_1$-$C_4$-haloalkyl or alkoxyalkyl having at most 4 carbon atoms.

3. A compound according to claim 1, wherein $R_1$ is the group $-NR_3R_4$, in which $R_3$ is hydrogen, methyl or ethyl, and $R_4$ is hydrogen, methyl, ethyl, methoxy, ethoxy or methoxymethyl.

4. A compound according to claim 1, wherein $R_2$ is 1,1,2,2-tetrafluoroethoxy.

5. A compound according to claim 1, wherein $R_2$ is $-G-CHF_2$, in which G is oxygen.

6. A compound according to claim 4, wherein $R_1$ is fluorine, chlorine, methyl, ethyl, methoxy, ethoxy, difluoromethoxy, trifluoromethyl, fluoromethyl, methoxymethyl or $-NR_3R_4$, in which $R_3$ is hydrogen or methyl, and $R_4$ is methyl or ethyl.

7. A compound according to claim 5, wherein E is the methine group, $R_1$ is fluorine, chlorine, methyl, ethyl, methoxy, ethoxy, difluoromethoxy, trifluoromethyl, fluoromethyl, methoxymethyl or $-NR_3R_4$, in which $R_3$ is hydrogen or methyl, and $R_4$ is methyl or ethyl.

8. A compound according to claim 7, wherein $R_1$ is methyl, ethyl, methoxy, ethoxy, difluoromethoxy, dimethylamino or ethylmethylamino.

9. A process for the preparation of a compound of the formula I according to claim 1, which process comprises reacting an amino compound of the formula II

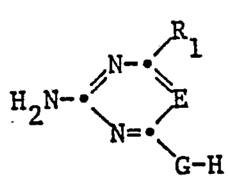 (II)

wherein E and $R_1$ are as defined for formula I, and G is oxygen, in the presence of a base, with difluorochloromethane, tetrafluoroethylene, trifluorochloroethylene, trifluorobromoethylene, hexafluoropropylene or difluorobromomethane.

10. Use of a fluoroalkoxy-aminopyrimidine or-triazine of the formula I according to claim 1 for preparing herbicides of the sulfonylurea class.

11. An N-fluoroalkoxypyrimidinyl- or -triazinylcarbamate of the general formula VII

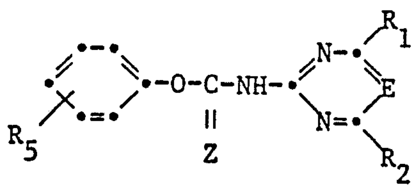

(VII)

wherein $R_1$, $R_2$ and E are as defined for formula I in claim 1, Z is oxygen or sulfur, and $R_5$ is a substituent from the series comprising hydrogen, halogen, nitro and $C_1$-$C_3$-alkyl.

**Revendications**

1 - Fluoralcoxy-aminopyrimidines et -triazines de formule générale I

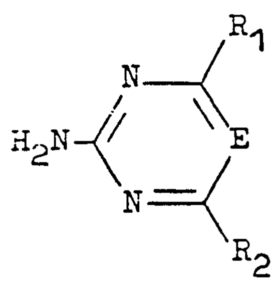

(I) ,

dans laquelle

E représente l'azote ou le groupe méthine,

$R_1$ représente un halogène, un groupe alkyle en C 1-C 4, alcoxy en C 1-C 4, halogénoalcoxy en C 1-C 4, alkylthio en C 1-C 4, halogénoalkyle en C 1-C 4, $-NR_3R_4$ ou alcoxyalkyle contenent au maximum 4 atomes de carbone, et

$R_2$ représente un groupe $-G-CF_2T$

dans lequel G représente l'oxygène et T l'hydrogène, un groupe -CHClF, -CHBrF, $-CHF_2$ ou $-CHF-CF_3$, $R_3$ représente l'hydrogène, un groupe méthyle ou éthyle et $R_4$ représente l'hydrogène, un groupe méthyle, éthyle, méthoxy, éthoxy ou méthoxyméthyle, avec la restriction que $R_1$ ne peut représenter un groupe alkyle en C 1-C 4 lorsque simultanément E représente l'azote.

2 - Composés selon la revendication 1, caractérisés en ce que $R_1$ représente un halogène, un groupe alkyle en C 1-C 4, alcoxy en C 1-C 4, halogénoalcoxy en C 1-C 4, alkylthio en C 1-C 4, halogénoalkyle en C 1-C 4 ou alcoxyalkyle contenant au maximum 4 atomes de carbone.

3 - Composés selon la revendication 1, caractérisés en ce que $R_1$ représente le groupe $-NR_3R_4$ dans lequel $R_3$ représente l'hydrogène, un groupe méthyle ou éthyle et $R_4$ l'hydrogène, un groupe méthyle, éthyle, méthoxy, éthoxy ou méthoxyméthyle.

4 - Composés selon la revendication 1, caractérisés en ce que $R_2$ représente un groupe 1,1,2,2-tétrafluoréthoxy.

5 - Composés selon la revendication 1, caractérisés en ce que $R_2$ représente un groupe $-G-CHF_2$ dans lequel G représente l'oxygène.

6 - Composés selon la revendication 4, caractérisés en ce que $R_1$ représente le fluor, le chlore, un groupe méthyle, éthyle, méthoxy, éthoxy, difluorométhoxy, trifluorométhyle, fluorométhyle, méthoxyméthyle ou $-NR_3R_4$ dans lequel $R_3$ représente l'hydrogène ou un groupe méthyle et $R_4$ un groupe méthyle ou éthyle.

7 - Composés selon la revendication 5, caractérisés en ce que E représente le groupe méthine, $R_1$ représente le fluor, le chlore, un groupe méthyle, éthyle, méthoxy, éthoxy, difluorométhoxy, trifluorométhyle, fluorométhyle, méthoxyméthyle ou $-NR_3R_4$ dans lequel $R_3$ représente l'hydrogène ou un groupe méthyle et $R_4$ un groupe méthyle ou éthyle.

8 - Composés selon la revendication 7, caractérisés en ce que $R_1$ représente un groupe méthyle, éthyle, méthoxy, éthoxy, difluorométhoxy, diméthylamino ou éthylméthylamino.

9 - Procédé de préparation des composés de formule I selon la revendication 1, caractérisé en ce que l'on fait réagir un composé aminé de formule II

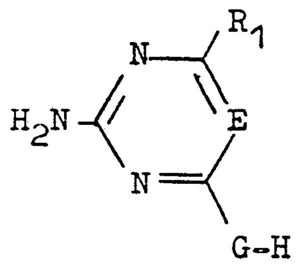

(II),

dans laquelle E et $R_1$ ont les significations indiquées en référence à la formule I et G représente l'oxygène, en présence d'une base, avec le difluorochlorométhane, le tétrafluoréthylène, le trifluorochloréthylène, le trifluorobrométhylène, l'hexafluoropropylène ou le difluorobromométhane.

10 - Utilisation des fluoralcoxy-aminopyrimidines et -triazines de formule I selon la revendication 1, pour la préparation d'herbicides de la classe des sulfonylurées.

11 - N-fluoralcoxypyrimidinyl- et -triazinylcarbamates de formule générale VII

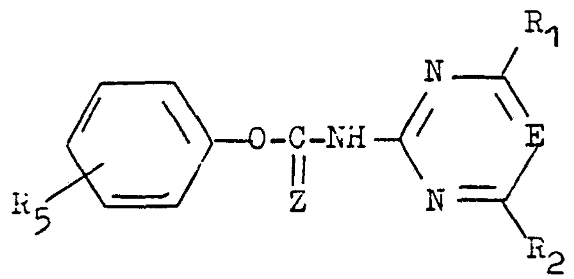

(VII)

dans laquelle $R_1$, $R_2$ et E ont les significations indiquées en référence à la formule I dans la revendication 1, Z représente l'oxygène ou le soufre et $R_5$ un substituant de la classe de l'hydrogène, des halogènes, des groupes nitro ou alkyle en C 1-C 3.